# EUROPEAN PATENT SPECIFICATION

(11) **EP 3 929 276 B1**
(45) Date of publication and mention of the grant of the patent: **04.10.2023**
(21) Application number: 20760137.8
(22) Date of filing: 03.02.2020
(51) Int. Cl.: C12M 1/34, G01N 21/64, C12N 15/09, C12Q 1/6825, G01N 33/53, G01N 33/542, C12Q 1/6818

(54) **NUCLEIC ACID SEQUENCE MEASUREMENT DEVICE, NUCLEIC ACID SEQUENCE MEASUREMENT METHOD, AND NUCLEIC ACID SEQUENCE MEASUREMENT APPARATUS**
VORRICHTUNG ZUR MESSUNG VON NUKLEINSÄURESEQUENZEN, VERFAHREN ZUR MESSUNG VON NUKLEINSÄURESEQUENZEN UND GERÄT ZUR MESSUNG VON NUKLEINSÄURESEQUENZEN
DISPOSITIF DE MESURE DE SÉQUENCE D'ACIDE NUCLÉIQUE, PROCÉDÉ DE MESURE DE SÉQUENCE D'ACIDE NUCLÉIQUE ET APPAREIL DE MESURE DE SÉQUENCE D'ACIDE NUCLÉIQUE

(30) Priority: 22.02.2019 JP 2019030752
(43) Date of publication of application: 29.12.2021
(73) Proprietor: Yokogawa Electric Corporation, Tokyo 180-8750 (JP)
(72) Inventor: MIYAUCHI, Yuki, Musashino-shi, Tokyo 180-8750 (JP); TADENUMA, Takashi, Musashino-shi, Tokyo 180-8750 (JP); TAGUCHI, Tomoyuki, Musashino-shi, Tokyo 180-8750 (JP)
(74) Representative: Grünecker Patent- und Rechtsanwälte PartG mbB
(86) International application number: PCT/JP2020/003856
(87) International publication number: WO 2020/170779

(56) References cited:
- EP-A1- 2 843 059
- WO-A1-2014/028061
- JP-A- 2004 016 132
- JP-A- 2013 514 803
- JP-B2- S5 928 906
- US-A1- 2004 009 514
- US-A1- 2011 281 740
- TADENUMA TAKASHI ET AL: "Development of a Nucleic Acid Detection System for Rapid Microbial Tests", YOKOGAWA TECHNICAL REPORT, vol. 60, no. 1, 1 January 2017 (2017-01-01), pages 7-12, XP055928497,

## Description

### [Technical Field]

The present invention relates to a nucleic acid sequence measurement device for measuring a target having a specific nucleic acid sequence contained in a sample by hybridization, a nucleic acid sequence measurement method, and a nucleic acid sequence measurement apparatus.

### [Background Art]

A nucleic acid sequence measurement device for measuring the presence and amount of a specific nucleic acid using a DNA chip is known (Patent Literature 1). In the nucleic acid sequence measurement device, when the binding between a fluorescent probe and quenching probe which are independent of each other is maintained via a binding part, the fluorescence of fluorescence molecules is quenched with quenching molecules. On the other hand, when a target is supplied, the target binds to a detection part, the binding is released via the binding part, quenching molecules separate from fluorescence molecules, and thus the fluorescence molecules exhibit fluorescence.

In addition, a light intensity measurement apparatus for measuring a light intensity of light captured by a light detection unit, including a light intensity calculation unit configured to calculate a light intensity of a fluorescence signal light based on an output signal of the light detection unit, an excitation light emission unit configured to emit excitation light, a reference fluorescence signal generation unit configured to generate a reference fluorescence signal by emitting this excitation light, and a calibration unit configured to calibrate a light intensity calculated by the light intensity calculation unit by applying light with a known light intensity with the reference fluorescence signal to the light detection unit is known (Patent Literature 2).

### [Citation List]

### [Patent Literature]

[Patent Literature 1]
   Japanese Patent No. 5928906
[Patent Literature 2]
   Japanese Unexamined Patent Application Publication No. 2011-17721

EP 2 843 059 A1 provides a nucleic acid sequence measuring method, a nucleic acid sequence measuring device, a manufacturing method for a nucleic acid sequence measuring device, and a nucleic acid sequence measuring apparatus. The nucleic acid sequence measuring device includes: a fluorescent probe having a coupling part and a base end and added with a fluorescent molecule at a predetermined position; a quenching probe having a coupling part and a base end and added with a quenching substance at a predetermined position; and a substrate having a solid surface to which the base end of each of the fluorescent probe and the quenching probe is fixed.

### [Summary of Invention]

### [Technical Problem]

However, in the method in Patent Literature 1, when the quenching with the binding between the fluorescent probe and the quenching probe is incomplete, since fluorescence (offset light) when there is no target is present, there is a constraint on the lower limit of detection due to the fluorescence light intensity of fluorescence molecules changing due to a small amount of target. In addition, in this method, the detection sensitivity is not sufficient because the change in fluorescence light intensity of only the fluorescent probe is detected.

In addition, the offset light is fluorescence of fluorescence molecules that are not quenched with quenching molecules while the fluorescent probe and the quenching probe are bound when hybridization with the target has not occurred. The binding between the fluorescent probe and the quenching probe is released by hybridization with the target, and the offset light decreases as the number of fluorescence molecules separated from quenching molecules increases. However, in the method in Patent Literature 1, it is not possible to measure this offset light.

On the other hand, in the light intensity measurement apparatus in Patent Literature 2, when comparing the light intensity before and after hybridization of the DNA chip, it is necessary to acquire an image of the DNA chip obtained by hybridizing a sample as a negative control that does not contain a target and an image of the DNA chip obtained by hybridizing a sample containing a target. Therefore, comparing the detected light intensities of the images of the DNA chip, there is a variation in the light intensity between chips and between spots, and the variation in the light intensity is a constraint on the lower limit of detection.

In addition, in the light intensity measurement apparatus, comparing the light intensities before and after hybridization of the same chip, it is necessary to acquire an image of the DNA chip before hybridization, promote a hybridization reaction by transferring the DNA chip temporarily from a biochip reading apparatus to an incubator, and then acquire an image of the DNA chip with this apparatus again, and this operation is complicated.

In addition, in order to acquire a fluorescence image of a fluorescent substance by excitation of a two-color laser using the light intensity measurement apparatus, since it is necessary to change the configuration of the apparatus when a fluorescence image of each fluorescent substance is acquired, there is a time lag in the acquisition of the two-color fluorescence images, and the correlation between the two-color fluorescence images may not be able to be obtained correctly.

An object of the present invention is to provide a nucleic acid sequence measurement device, a nucleic acid sequence measurement method, and a nucleic acid sequence measurement apparatus which are not affected by variations in the light intensity between chips and between spots and have excellent detection sensitivity.

### [Solution to Problem]

In order to achieve the above object, the present invention provides the following configuration.
[1] A nucleic acid sequence measurement device that measures a target having a specific nucleic acid sequence included in a sample by means of hybridization, the nucleic acid sequence measurement device including:
   a donor fluorescent probe having a first binding part and a first base end and having a donor fluorescent molecule added at predetermined position;
   a quenching probe having a second binding part and a second base end and having an acceptor fluorescent molecule added at predetermined position; and
   a substrate having a solid phase surface to which the first base end of the donor fluorescent probe and the second base end of the quenching probe are fixed,
   wherein the first binding part of the donor fluorescent probe and the second binding part of the quenching probe have sequences complementary to each other,
   wherein at least one of the donor fluorescent probe and the quenching probe has a detection part having a sequence complementary to a nucleic acid sequence of the target, and
   wherein the donor fluorescent probe and the quenching probe have a positional relationship in which fluorescence of the donor fluorescent molecule is quenched with the acceptor fluorescent molecule that approaches the donor fluorescent molecule, and the first base end and the second base end are fixed to the solid phase surface.
[2] The nucleic acid sequence measurement device according to [1],
   wherein, when hybridization between the target and the detection part has not occurred, a binding between the first binding part of the donor fluorescent probe and the quenching probe is maintained, and thus the fluorescence of the donor fluorescent molecule is quenched with the acceptor fluorescent molecule that approaches the donor fluorescent molecule, and the acceptor fluorescent molecule exhibits fluorescence, and
   wherein, when hybridization between the target and the detection part occurs, the binding between the first binding part of the donor fluorescent probe and the second binding part of the quenching probe is released, the donor fluorescent molecule separated from the acceptor fluorescent molecule exhibits fluorescence.
[3] The nucleic acid sequence measurement device according to [1] or [2], wherein the donor fluorescent probe has the detection part.
[4] The nucleic acid sequence measurement device according to [1] or [2], wherein the quenching probe has the detection part.
[5] The nucleic acid sequence measurement device according to any one of [1] to [4], wherein the substrate is a flat plate, and the solid phase surface is a flat surface of the flat plate.
[6] The nucleic acid sequence measurement device according to any one of [1] to [3] and [5], wherein at least a part of the first binding part of the donor fluorescent probe functions as the detection part.
[7] The nucleic acid sequence measurement device according to any one of [1], [2], [4] and [5], wherein at least a part of the second binding part of the quenching probe functions as the detection part.
[8] The nucleic acid sequence measurement device according to any one of [1] to [7], wherein the predetermined position to which the donor fluorescent molecule is added is in the middle of the donor fluorescent probe.
[9] The nucleic acid sequence measurement device according to any one of [1] to [7], wherein the predetermined position to which the acceptor fluorescent molecule is added is in the middle of the quenching probe.
[10] The nucleic acid sequence measurement device according to any one of [1] to [9], wherein both the donor fluorescent probe and the quenching probe have the detection part.
[11] A nucleic acid sequence measurement method of measuring a target having a specific nucleic acid sequence included in a sample by means of hybridization, the nucleic acid sequence measurement method including:
   (a) a step of preparing a sample containing the target;
   (b) a step of supplying the sample to a nucleic acid sequence measurement device;
   (c) a step of measuring a fluorescence amount of a donor fluorescent molecule and a fluorescence amount of an acceptor fluorescent molecule from the nucleic acid sequence measurement device;
   (d) a step of causing a hybridization reaction between the target in the sample and at least one of a donor fluorescent probe or a quenching probe in the nucleic acid sequence measurement device; and
   (e) a step of measuring a fluorescence amount of the donor fluorescent molecule and a fluorescence amount of the acceptor fluorescent molecule from the nucleic acid sequence measurement device after the reaction,
   wherein the nucleic acid sequence measurement device includes:
   a donor fluorescent probe having a first binding part and a first base end and having the donor fluorescent molecule added at a predetermined position;
   a quenching probe having a second binding part and a second base end and having the acceptor fluorescent molecule added at a predetermined position; and
   a substrate having a solid phase surface to which the first base end of the donor fluorescent probe and the second base end of the quenching probe are fixed,
   wherein the first binding part of the donor fluorescent probe and the second binding part of the quenching probe have sequences complementary to each other,
   wherein at least one of the donor fluorescent probe and the quenching probe has a detection part having a sequence complementary to a nucleic acid sequence of the target, and
   wherein the donor fluorescent probe and the quenching probe have a positional relationship in which fluorescence of the donor fluorescent molecule is quenched with the acceptor fluorescent molecule that approaches the donor fluorescent molecule, and the first base end and the second base end are fixed to the solid phase surface.
[12] The nucleic acid sequence measurement method according to [11], further including:
   calculating a number of molecules of the hybridized target from a change in the fluorescence amount of the donor fluorescent molecule between before and after the hybridization reaction.
[13] The nucleic acid sequence measurement method according to [11], further including:
   calculating a number of molecules of the target that are not hybridized from a change in the fluorescence amount of the acceptor fluorescent molecule between before and after the hybridization reaction.
[14] A nucleic acid sequence measurement apparatus including:
   the nucleic acid sequence measurement device according to any one of [1] to [10], and
   a fluorescence reading apparatus configured to measure a fluorescence amount of the donor fluorescent molecule and a fluorescence amount of the acceptor fluorescent molecule from the nucleic acid sequence measurement device.
[15] The nucleic acid sequence measurement apparatus according to [14], wherein, in a presence of the sample, a fluorescence amount of the donor fluorescent molecule and a fluorescence amount of the acceptor fluorescent molecule from the nucleic acid sequence measurement device are measured.
[16] The nucleic acid sequence measurement apparatus according to [14] or [15], further including a stirring function for hybridizing the target with the donor fluorescent probe or the quenching probe.
[17] The nucleic acid sequence measurement apparatus according to any one of [14] to [16], further including a function of separating the fluorescence from the nucleic acid sequence measurement device into two colors and measuring the fluorescence at the same time.

### [Advantageous Effects of Invention]

According to the nucleic acid sequence measurement device of the present invention, the amount of the probe set in which the binding is maintained can be measured by measuring the fluorescence of acceptor fluorescent molecules. In addition, offset light of fluorescence of the donor fluorescent molecules can be detected and quantified as fluorescence of the acceptor fluorescent molecules. Therefore, the offset light of donor fluorescent molecules can be subtracted according to computation according to the fluorescence of the acceptor fluorescent molecules, and the change in fluorescence of the donor fluorescent molecules due to hybridization with the target can thus be detected with high accuracy.

In addition, it is possible to capture the decrease in fluorescence of acceptor fluorescent molecules, which has a negative correlation with the increase in fluorescence of donor fluorescent molecules due to progress of the hybridization reaction with the target, and double the correlation to improve the detection sensitivity.

In addition, the lower limit of detection can be lowered without being affected by variations in the light intensity between chips and between spots.

According to the nucleic acid sequence measurement method of the present invention, the amount of the probe set in which the binding is maintained can be measured by measuring fluorescence of acceptor fluorescent molecules. In addition, offset light of fluorescence of the donor fluorescent molecules can be detected and quantified as fluorescence of the acceptor fluorescent molecules. Therefore, the offset light of donor fluorescent molecules can be subtracted according to computation according to the fluorescence of the acceptor fluorescent molecules, and the change in fluorescence of the donor fluorescent molecules due to hybridization with the target can thus be detected with high accuracy.

In addition, it is possible to capture the decrease in fluorescence of acceptor fluorescent molecules, which has a negative correlation with the increase in fluorescence of donor fluorescent molecules due to progress of the hybridization reaction with the target, and double the correlation to improve the detection sensitivity.

In addition, the lower limit of detection can be lowered without being affected by variations in the light intensity between chips and between spots.

In addition, according to the nucleic acid sequence measurement method of the present invention, a labeling process is not necessary, and a washing process is also omitted. Therefore, the time and effort for a hybridization experiment are further reduced, and an operation time and cost are reduced. In addition, it is possible to avoid performance deterioration, a decrease in the light intensity, an increase in background light, the occurrence of variation or the like due to an inadequate washing process. In the conventional method, there is a risk of increase and variation in signals and background light due to the washing method, a washing degree, and non-uniform washing or the like, but such a risk can be avoided according to the present invention. Therefore, more uniform results can be obtained on the array surface, and the reproducibility of detection is also improved.

In addition, according to the nucleic acid sequence measurement method of the present invention, real time observation during hybridization is possible. That is, while a solution containing detection target molecules (target) is added to a DNA array (wet condition), the array can be observed. Therefore, the light intensity can be checked when the influence of washing is excluded, and real time observation during hybridization is possible. Therefore, in some situations such as when the sample concentration is high and hybridization proceeds quickly, the hybridization can be completed in a shorter time.

According to the nucleic acid sequence measurement apparatus of the present invention, at the same position coordinates in the spot of the DNA chip, a change in the light intensity between before and after the hybridization reaction at the same time can be calculated according to computation, and thus the variation in the probe fixed in the spot and the variation in the hybridization reaction can be determined in detail. In addition, in the fluorescence image, pixels having a large correlation between the changes in the fluorescence amount of the donor fluorescent molecules (hereinafter referred to as a donor fluorescence amount) and the fluorescence amount of the acceptor fluorescent molecules (hereinafter referred to as an acceptor fluorescence amount) can be selected, the change in the light intensity can be calculated according to computation, and measurement with high accuracy is possible.

In a conventional nucleic acid sequence measurement apparatus, since the average light intensity of the entire spot is calculated according to computation and the change in the light intensity is confirmed, when there is unevenness in the amount of probe molecules fixed in the surface distribution of spots or unevenness in the change in the light intensity due to the hybridization reaction, the part in which the change in the light intensity due to the hybridization reaction in the spot is observed is overlooked. According to the nucleic acid sequence measurement apparatus of the present invention, since it is possible to acquire the image of the spot, the change in the light intensity can be confirmed for each pixel of a detector, and a detailed change in the light intensity can be detected. In addition, when pixels having a large correlation between the changes in the donor fluorescence amount and the acceptor fluorescence amount are selected, it is possible to accurately detect whether there is a change in the light intensity due to the hybridization reaction. Therefore, it is possible to acquire a fluorescence image (donor fluorescence image) of donor fluorescent molecules and a fluorescence image (acceptor fluorescence image) of acceptor molecules with no positional deviation at the same timing, and more accurate analysis is possible.

### [Brief Description of Drawings]

FIG. 1 is a diagram showing a configuration example of a probe.
FIG. 2 is a diagram schematically showing a principle for detecting a target.
FIG. 3 is a diagram showing a modified example and is a diagram showing an example in which a donor fluorescent molecule and an acceptor fluorescent molecule are positioned in the middle of a probe.
FIG. 4A is a diagram showing a modified example and is a diagram showing an example in which donor fluorescent molecules and acceptor fluorescent molecules are added to a plurality of locations.
FIG. 4B is a diagram showing a modified example and is a diagram showing an example in which a target binds to a quenching probe.
FIG. 5 is a configuration diagram showing a nucleic acid sequence measurement apparatus.
FIG. 6 is a schematic diagram of a donor fluorescence image and an acceptor fluorescence image separated by wavelength.
FIG. 7 is a diagram showing a change in light intensity of a hybridization reaction when targets with different concentrations are hybridized.
FIG. 8 is a diagram showing a corrected light intensity obtained by setting a coefficient to multiply a fluorescence amount of the acceptor fluorescent molecule so that a fluorescence amount of the acceptor fluorescent molecules when there is no target is equal to a fluorescence amount of the donor fluorescent molecules, and subtracting a numerical value obtained by multiplying a fluorescence amount of the acceptor fluorescent molecules of targets with different concentrations by the set coefficient from the fluorescence amount of the donor fluorescent molecules.

### [Description of Embodiments]

A nucleic acid sequence measurement device of the present invention is a nucleic acid sequence measurement device for measuring a target having a specific nucleic acid sequence contained in a sample by hybridization, including
a donor fluorescent probe having a first binding part and a first base end and having donor fluorescent molecules added at predetermined positions,
a quenching probe having a second binding part and a second base end and having acceptor fluorescent molecules added at predetermined positions, and
a substrate having a solid phase surface to which the first base end of the donor fluorescent probe and the second base end of the quenching probe are fixed,
wherein the first binding part of the donor fluorescent probe and the second binding part of the quenching probe have sequences complementary to each other,
wherein at least one of the donor fluorescent probe and the quenching probe has a detection part having a sequence complementary to a nucleic acid sequence of the target, and
wherein the donor fluorescent probe and the quenching probe have a positional relationship in which fluorescence of the donor fluorescent molecules is quenched with the acceptor fluorescent molecules that approach the donor fluorescent molecules, and the first base end and the second base end are fixed to the solid phase surface.

Hereinafter, a nucleic acid sequence measurement device according to an embodiment the present invention will be described.

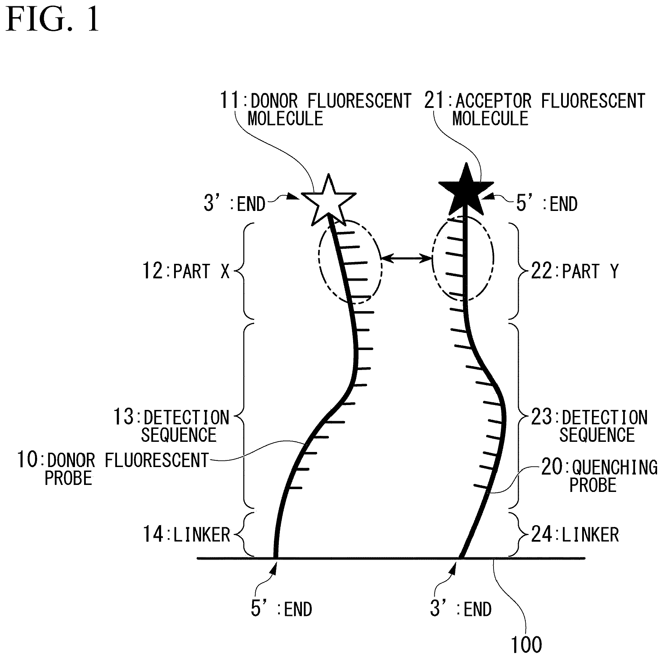

FIG. 1 is a diagram showing a configuration example of a probe.

As shown in FIG. 1, a nucleic acid sequence measurement device of the present embodiment has a configuration in which a donor fluorescent probe 10 in which donor fluorescent molecules 11 are added to a complementary sequence of a target 30 which is a nucleic acid as a detection target and a quenching probe 20 to which acceptor fluorescent molecules 21 are added are fixed to a solid phase surface 100 such as a substrate. In the present invention, a principle for fluorescence resonance energy transfer (FRET) from the donor fluorescent molecule 11 to the acceptor fluorescent molecule 21 is used, and when the acceptor fluorescent molecule 21 approaches the donor fluorescent molecule 11, energy is transferred from the donor fluorescent molecule 11 to the acceptor fluorescent molecule 21 due to excitation of the donor fluorescent molecule 11, and acceptor fluorescent molecules exhibit fluorescence.

The combination of donor fluorescent molecules and acceptor fluorescent molecules is not particularly limited as long as it is a combination in which fluorescence resonance energy transfer occurs, but combination of donor fluorescent molecules and acceptor fluorescent molecules, which has high fluorescence resonance energy transfer efficiency, is preferable. When the combination of donor fluorescent molecules and acceptor fluorescent molecules, which has high fluorescence resonance energy transfer efficiency, is selected, it is possible to further improve the sensitivity. Table 1 shows an example of combinations of donor fluorescent molecules and acceptor fluorescent molecules in which energy transfer occurs, and shows the excitation wavelength and the fluorescence wavelength of fluorescence molecules. In the following Table 1, the combinations of two types of fluorescence molecules on the left and right of each row of donor fluorescent molecules and acceptor fluorescent molecules show examples of combinations of donor fluorescent molecules and acceptor fluorescent molecules.

**[Table 1]**

| Donor fluorescent molecule | | | Acceptor fluorescent molecule | | |
|---|---|---|---|---|---|
| Pigment name | Excitation wavelengt h (nm) | Fluorescenc e wavelength (nm) | Pigment name | Excitation wavelengt h (nm) | Fluorescenc e wavelength (nm) |
| Alexa Fluor 488 | 495 | 519 | Alexa Fluor 555 | 555 | 565 |
| Alexa Fluor 488 | 495 | 519 | Cy3 | 550 | 570 |
| Alexa Fluor 488 | 495 | 519 | Alexa Fluor 546 | 556 | 573 |
| Alexa Fluor 488 | 495 | 519 | Alexa Fluor 647 | 650 | 665 |
| Fluorescein | 494 | 521 | ATTO 655 | 663 | 684 |
| FITC | 498 | 522 | Cy3 | 550 | 570 |
| FITC | 498 | 522 | Rhodamin e | 550 | 570 |
| ATTO 532 | 532 | 553 | Alexa Fluor 647 | 650 | 665 |
| Cy3 | 550 | 570 | Cy5 | 643 | 667 |
| Cy3 | 550 | 570 | Cy5.5 | 675 | 694 |
| PE | 488 | 578 | Cy5.5 | 675 | 694 |
| R-PE | 566 | 578 | Alexa Fluor 610 | 612 | 628 |
| R-PE | 566 | 578 | Alexa Fluor 647 | 650 | 665 |
| R-PE | 566 | 578 | Cy5.5 | 675 | 694 |
| R-PE | 566 | 578 | Alexa Fluor 680 | 684 | 707 |
| APC (Allophycocyani n) | 650 | 660 | Alexa Fluor 700 | 702 | 723 |
| APC (Allophycocyani n) | 650 | 660 | Alexa Fluor 750 | 749 | 782 |

As shown in FIG. 1, the donor fluorescent probe 10 includes a part X 12, a detection sequence 13, and a linker 14. The part X 12 is provided from the 3' end and is a part of several bases as a complementary sequence of the target 30. The detection sequence 13 is provided following the part X 12 and is a complementary sequence of the target 30. The linker 14 is connected to the detection sequence 13 and continues to the 5' end. The donor fluorescent molecule 11 is fixed at the 3' end of the donor fluorescent probe 10.

The quenching probe 20 includes a part Y22, a detection sequence 23, and a linker 24. The part Y22 is a part of several bases provided from the 5' end. The detection sequence 23 is provided following the part Y22 and is a complementary sequence of the target 30. The linker 24 is connected to the detection sequence 23 and continues to the 3' end. The acceptor fluorescent molecule 21 is fixed to the 5' end of the quenching probe 20.

The donor fluorescent probe 10 and the quenching probe 20 are fixed to the solid phase surface 100 via the linker 14 and the linker 24. In addition, the sequence of the part X 12 of the donor fluorescent probe 10 is complementary to the sequence of the part Y22 of the quenching probe 20. In addition, the positional relationship in which the donor fluorescent probe 10 and the quenching probe 20 are fixed at positions at which the part X 12 of the donor fluorescent probe 10 and the part Y22 of the quenching probe 20 can bind to each other, and when the part X 12 of the donor fluorescent probe 10 and the part Y22 of the quenching probe 20 are bound, the acceptor fluorescent molecule 21 approaches the donor fluorescent molecule 11, and when excitation light is emitted to donor fluorescent molecules, energy is transferred from the donor fluorescent molecule 11 to the acceptor fluorescent molecule 21, acceptor fluorescent molecules exhibit fluorescence is secured.

Here, complementary in the present invention means that one nucleic acid sequence has a nucleic acid sequence that can form a double-stranded state with the other nucleic acid sequence, and they are not necessarily completely complementary, but may include several mismatched base pairs.

In addition, it is desirable to design the affinity between the donor fluorescent probe 10 and the target 30 to be higher than the affinity between the donor fluorescent probe 10 and the quenching probe 20 with the part X 12 and the part Y22.

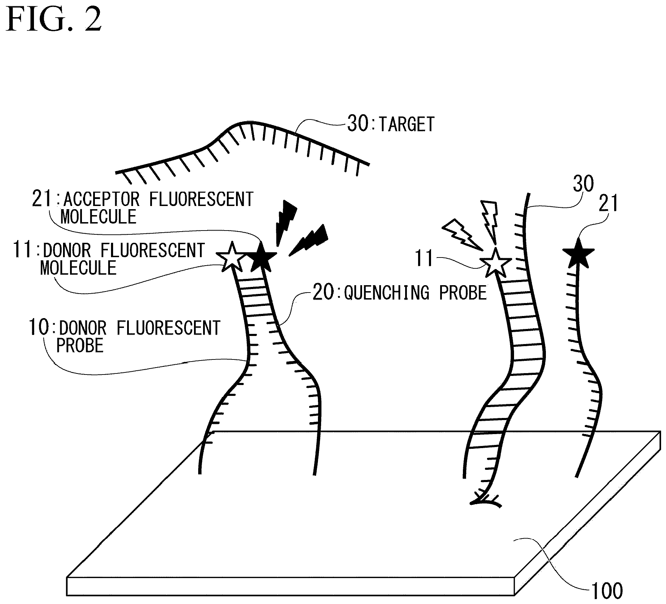

Next, a principle for detecting the target 30 using the nucleic acid sequence measurement device of the present invention will be described. FIG. 2 is a diagram schematically showing a principle for detecting a target.

As shown in FIG. 2, when the target 30 is not present, the donor fluorescent probe 10 to which the donor fluorescent molecules 11 are added binds to the quenching probe 20 to which the acceptor fluorescent molecules 21 are added. Therefore, the donor fluorescent molecule 11 and the acceptor fluorescent molecule 21 are in close contact with each other. When excitation light is emitted to donor fluorescent molecules in this state, the energy of the excited donor fluorescent molecule 11 is transferred to the acceptor fluorescent molecule 21, the donor fluorescent molecule 11 is quenched, and the acceptor fluorescent molecule 21 exhibits fluorescence.

When the target 30 is present, the target 30 binds to the donor fluorescent probe 10. When the target 30 binds to the donor fluorescent probe 10, the binding between the donor fluorescent probe 10 and the quenching probe 20 is released, and the distance between the acceptor fluorescent molecule 21 and the donor fluorescent molecule 11 increases. Therefore, transfer of energy from donor fluorescent molecules to acceptor fluorescent molecules is eliminated and acceptor fluorescent molecules do not exhibit fluorescence, and when excitation light is emitted to donor fluorescent molecules, the donor fluorescent molecule 11 exhibits fluorescence. Therefore, when the solid phase surface 100 is observed with a fluorescence reading apparatus, it is possible to confirm whether there is a target nucleic acid (the target 30) in the sample according to whether the donor fluorescent probe 10 exhibits fluorescence.

A nucleic acid sequence measurement method of the present invention is not limited to the above embodiment, and various modifications can be made as follows.

When the donor fluorescent probe to which donor fluorescent molecules are added and the quenching probe to which acceptor fluorescent molecules are added are fixed while changing their abundance ratio, it is possible to control the fluorescence amount when there are no target molecules. For example, when the number of quenching probes is larger than the number of donor fluorescent probes, the probability of donor fluorescent molecules being coupled increases and the fluorescence amount of donor fluorescent molecules decreases. Therefore, the fluorescence (offset light) of donor fluorescent molecules when there are no target molecules can be reduced to a low level. In addition, when the number of donor fluorescent probes is larger than the number of quenching probes, the probability of transfer of energy to acceptor fluorescent molecules occurring is low, and the fluorescence (hybridization light intensity) exhibited after a target substance is detected becomes stronger.

In the above embodiment, the sequence of the part X 12 of the donor fluorescent probe 10 is complementary to the target 30, but the sequences of the part X 12 of the donor fluorescent probe 10 and the part Y22 of the quenching probe 20 may be a common sequence regardless of the type of the target. In this case, since the part X 12 and the part Y22 have the same structure regardless of the type of the target, and only the detection sequence 13 and the detection sequence 23 need to be changed according to the type of the target, the design becomes easy. In addition, there is an advantage that quenching/light emitting characteristics are constant regardless of the detection target.

In addition, the solid phase surface to which the donor fluorescent probe and the quenching probe are fixed is not limited to the plane on the substrate. The donor fluorescent probe and the quenching probe may be fixed to the surface of beads. When the donor fluorescent probe and the quenching probe are fixed to the surface of beads, the donor fluorescent probe and the quenching probe have a shape that spreads radially around the beads. In this case, the surface area of the solid phase surface to which the probe is fixed is larger, and the amount of the probe per unit area can increase. In addition, when beads in which detection target molecules are collected are recovered according to their size, magnetism or the like, detection target molecules can be selectively recovered. The recovered molecules can be used for another test in a subsequent process.

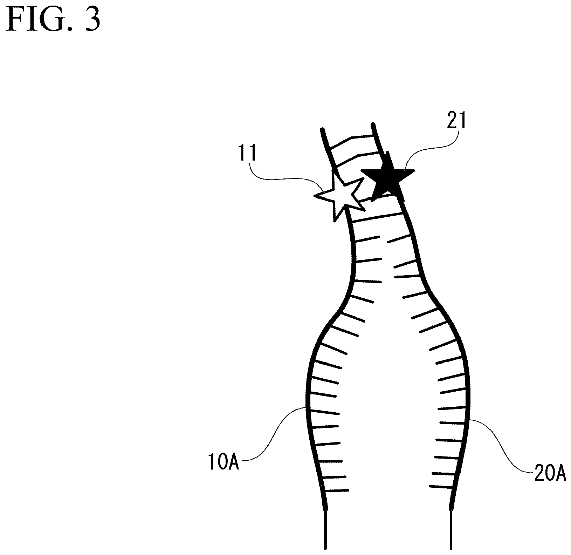

The donor fluorescent molecules or acceptor fluorescent molecules may not be attached to the tip of the probe. FIG. 3 shows an example in which donor fluorescent molecules and acceptor fluorescent molecules are positioned in the middle of a probe. In the example in FIG. 3, the donor fluorescent molecules 11 are added in the middle of a donor fluorescent probe 10A, and the acceptor fluorescent molecules 21 are added in the middle of a quenching probe 20A. However, when the donor fluorescent probe 10A and the quenching probe 20A bind to each other so that transfer of energy from the donor fluorescent molecule 11 to the acceptor fluorescent molecule 21 occurs, it is desirable to design positions at which the acceptor fluorescent molecules 21 face the donor fluorescent molecules 11 so that they are adjacent to each other. When donor fluorescent molecules or acceptor fluorescent molecules are added at positions other than the tip of the probe, there is an advantage that the tip of the probe can be additionally modified.

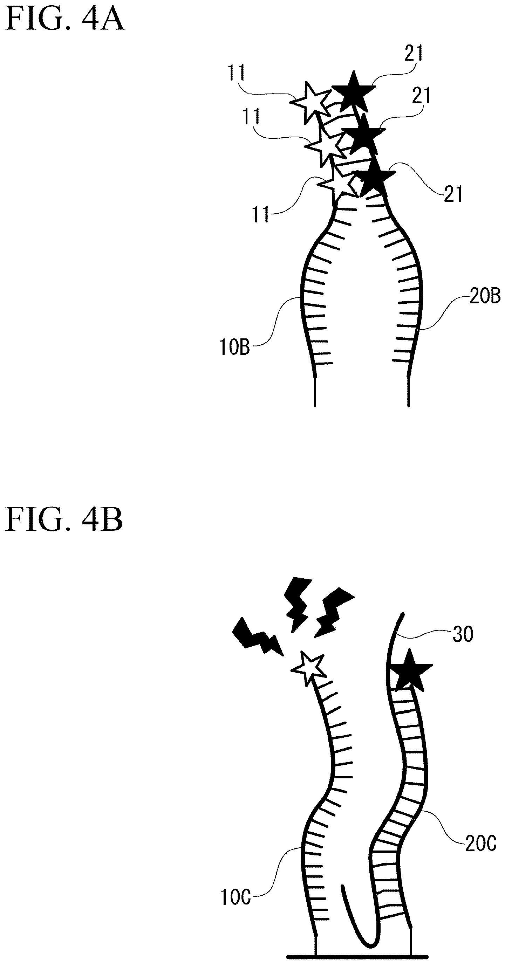

Donor fluorescent molecules and acceptor fluorescent molecules may be added to a plurality of types and at a plurality of locations. FIG. 4A is a diagram showing an example in which donor fluorescent molecules and acceptor fluorescent molecules are added at a plurality of locations. In the example in FIG. 4A, the donor fluorescent molecules 11, 11, and 11 are added to a donor fluorescent probe 10B and the acceptor fluorescent molecules 21, 21, and 21 are added to a quenching probe 20B. When a plurality of donor fluorescent molecules and acceptor fluorescent molecules are added to one probe, the types of donor fluorescent molecules or acceptor fluorescent molecules may be different. When a plurality of donor fluorescent molecules and acceptor fluorescent molecules are added to one probe, the fluorescence amount of the acceptor fluorescent molecules when the target 30 does not bind and the fluorescence amount of the donor fluorescent molecules when the target binds increase, and can be detected with higher sensitivity. In addition, when the fluorescence amount of the donor fluorescent molecules when there are no target molecules is large and the fluorescence becomes offset light for detecting fluorescence of acceptor fluorescent molecules, molecules that absorb light in a detection wavelength of fluorescence of acceptor fluorescent molecules which is in a wavelength range not covering an absorption wavelength spectrum of acceptor fluorescent molecules within a fluorescence wavelength spectrum of donor fluorescent molecules may be added as donor fluorescent molecules to the donor fluorescent probe. These molecules may be added as acceptor fluorescent molecules to the quenching probe. Therefore, the fluorescence (offset light) of donor fluorescent molecules when there are no target molecules can be reduced to a low level. In addition, when the fluorescence amount of the acceptor fluorescent molecules when there are target molecules is large and the fluorescence becomes offset light for detecting fluorescence of donor fluorescent molecules, molecules that absorb light in a wavelength range of an excitation light source so that excitation light does not directly excite acceptor fluorescent molecules may be added as acceptor fluorescent molecules to the quenching probe. Therefore, the fluorescence (offset light) of acceptor fluorescent molecules when there are target molecules can be reduced to a low level.

In the above embodiment, not only the detection sequence 13 of the donor fluorescent probe 10, but also the detection sequence 23 is provided for the quenching probe 20. However, the detection sequence complementary to the target may be provided for only the donor fluorescent probe. However, it is though that, when the detection sequence is provided for both probes, it is possible to increase the binding frequency of the target.

In addition, in the above embodiment, the target 30 is designed so that it binds to the donor fluorescent probe 10, but the target may be designed so that it binds to the quenching probe. In this case, it is possible to avoid a change in characteristics of the donor fluorescent molecules due to the target that approaches.

FIG. 4B is a diagram showing an example in which the target 30 binds to a quenching probe 20C. As shown in the example in FIG. 4B, a sequence that has higher affinity with respect to the target 30 is provided for the quenching probe 20C, and the target 30 may bind to the quenching probe 20C instead of a donor fluorescent probe 10C. In this case, a detection sequence that is complementary to the target may or may not be provided for the donor fluorescent probe 10C.

Next, a method of producing a nucleic acid sequence measurement device of the present invention will be described.

### (1) Preparation of solution

First, a probe solution in which the donor fluorescent probe 10 and the quenching probe 20 are mixed is prepared, and the concentration of the probe is adjusted.

### (2) Coupling

Next, the probe solution is heated, and then rapidly cooled, and the donor fluorescent probe 10 and the quenching probe 20 are coupled. Thereby, the donor fluorescent probe 10 and the quenching probe 20 are bound via the part X 12 and the part Y22. Here, for example, the probe solution is heated at 95°C and the temperature was then maintained for 5 minutes, rapid cooling to 25°C was then performed, and the donor fluorescent probe 10 and the quenching probe 20 were coupled.

### (3) Fixing to solid phase surface

Next, the probe solution in which the donor fluorescent probe 10 and the quenching probe 20 are coupled is spotted on the solid phase surface, and the donor fluorescent probe 10 and the quenching probe 20 are fixed to the solid phase surface 100.

### (4) Washing

Next, the solid phase surface 100 is washed and an excess unfixed probe is removed. A DNA chip is produced according to the above procedures.

In this manner, the donor fluorescent probe 10 and the quenching probe 20 that are bound to each other via the part X 12 and the part Y22 bind to the solid phase surface 100. Therefore, the positional relationship between the donor fluorescent probe 10 and the quenching probe 20 can be appropriately managed, and transfer of energy from donor fluorescent molecules to acceptor fluorescent molecules can be appropriately performed. Therefore, the detection sensitivity can be improved.

Next, a nucleic acid sequence measurement method using a nucleic acid sequence measurement device of the present invention will be described.

The nucleic acid sequence measurement method of the present invention is a nucleic acid sequence measurement method using the nucleic acid sequence measurement device of the present invention, and is a nucleic acid sequence measurement method of measuring a target having a specific nucleic acid sequence contained in a sample by hybridization.

The nucleic acid measurement method of the present invention includes the following steps (a) to (e).
(a) a step of preparing a sample containing the target,
(b) a step of supplying the sample to a nucleic acid sequence measurement device,
(c) a step of measuring a fluorescence amount of donor fluorescent molecules and a fluorescence amount of acceptor fluorescent molecules from the nucleic acid sequence measurement device of the present invention,
(d) a step of causing a hybridization reaction between the target in the sample and at least one of a donor fluorescent probe or a quenching probe in the nucleic acid sequence measurement device, and
(e) a step of measuring a fluorescence amount of the donor fluorescent molecules and a fluorescence amount of the acceptor fluorescent molecules from the nucleic acid sequence measurement device after the reaction.

The steps will be described below.

First, a sample 50 containing a target 30 having a desired specific nucleic acid sequence is prepared (step (a)). In the preparation of the sample 50, a gene having a specific nucleic acid sequence (the target 30) may be amplified.

At a stage in which a gene is amplified, a test for confirming whether a gene is amplified is performed, and only when a gene is amplified, a hybridization reaction to be described below may be performed.

Here, a timing at which it is examined whether there is a gene is not limited to a timing after amplification is completed, but may be during the amplification reaction. As an examination method, electrophoresis, an antigen-antibody reaction, mass spectrometry, a real-time PCR method, and the like can be appropriately used.

In addition, the nucleic acid (the target 30) may be bound to proteins, sugar chains, or the like. In this case, the interaction of proteins, sugar chains or the like with the nucleic acid (the target 30) can be confirmed.

Next, the sample 50 containing the target 30 is supplied to the solid phase surface 100 of the nucleic acid measurement device (step (b)). Then, a fluorescence amount of the donor fluorescent molecules and a fluorescence amount of the acceptor fluorescent molecules from the nucleic acid sequence measurement device are measured (step (c)).

As described above, when the target 30 is not present, the donor fluorescent probe 10 to which the donor fluorescent molecules 11 are added binds to the quenching probe 20 to which the acceptor fluorescent molecules 21 are added, the donor fluorescent molecule 11 and the acceptor fluorescent molecule 21 are in close contact with each other. When excitation light is emitted to donor fluorescent molecules in this state, the energy of the excited donor fluorescent molecule 11 is transferred to the acceptor fluorescent molecule 21, the donor fluorescent molecule 11 is quenched, and the acceptor fluorescent molecule 21 exhibits fluorescence.

However, even if the donor fluorescent probe 10 and the quenching probe 20 are bound, in the acceptor fluorescent molecule 21 of the quenching probe 20, the fluorescence of the donor fluorescent molecule 11 of the donor fluorescent probe 10 may not be completely quenched. This unquenched fluorescence becomes offset light of the donor fluorescent molecule 11, and this offset light can be regarded as the fluorescence amount of the acceptor fluorescent molecules 21 when the hybridization reaction between the target 30 and at least one of the donor fluorescent probe 10 and the quenching probe 20 does not occur. Therefore, a fluorescence amount of the donor fluorescent molecules 11 and a fluorescence amount of the acceptor fluorescent molecules before and after the hybridization reaction can be measured, and the amount of offset light can be calculated according to computation from the fluorescence amount of the donor fluorescent molecules 11 and the fluorescence amount of the acceptor fluorescent molecules 21. Therefore, the number of molecules hybridized before and after the hybridization reaction can be calculated more accurately.

Next, the target 30 in the sample 50 and at least one of the donor fluorescent probe and the quenching probe in the nucleic acid sequence measurement device undergo a hybridization reaction (step (d)).

When the target 30 in the sample 50 and at least one of the donor fluorescent probe and the quenching probe in the nucleic acid sequence measurement device undergo a hybridization reaction, the target 30 binds to at least one of the donor fluorescent probe 10 and the quenching probe 20, the binding between the donor fluorescent probe 10 and the quenching probe 20 is released, and the distance between the acceptor fluorescent molecule 21 and the donor fluorescent molecule 11 increases. Therefore, transfer of energy from the donor fluorescent molecule 11 to the acceptor fluorescent molecule 21 is eliminated, the acceptor fluorescent molecules 21 do not exhibit fluorescence, and when excitation light is emitted to the donor fluorescent molecules 11, the donor fluorescent molecules 11 exhibit fluorescence.

Next, after the hybridization reaction, a fluorescence amount (donor fluorescence amount) of donor fluorescent molecules and a fluorescence amount (acceptor fluorescence amount) of the acceptor fluorescent molecules from the nucleic acid sequence measurement device are measured by a fluorescence reading apparatus 60 (step (e)).

With the fluorescence reading apparatus 60, it is possible to confirm whether there is a target nucleic acid (the target 30) in the sample according to whether the donor fluorescent probe 10 exhibits fluorescence, and the hybridized target nucleic acid (the target 30) can be quantified. In addition, at this time, since the uncollected target 30 contained in the solution does not exhibit fluorescence, washing is not necessary. Therefore, in the presence of a target solution, it is possible to observe the solid phase surface 100 of the nucleic acid sequence measurement device through the solution. Therefore, the light intensity can be measured when the influence of washing is excluded, and real time measurement during hybridization is also possible.

In addition, when the nucleic acid measurement apparatus of the present invention to be described below is used as the fluorescence reading apparatus, it is possible to acquire images before and after hybridization at the same coordinates and acquire a donor fluorescence image and an acceptor fluorescence image separated by wavelength at the same time. In addition, it is also possible to calculate the number of molecules of the hybridized target by analyzing the donor fluorescence image and the acceptor fluorescence image.

In addition, according to the nucleic acid sequence measurement method of the present invention, it is possible to calculate the number of target molecules 30 that have undergone a hybridization reaction from the fluorescence change amount of the donor fluorescent molecules 11 before and after the hybridization reaction. For example, a hybridization reaction is performed using a standard solution of the target molecules 30 having a known number of molecules, the fluorescence change amount of the donor fluorescent molecules 11 before and after the reaction is measured, and a calibration curve showing the relationship between the number of molecules and the fluorescence change amount is created in advance. From this calibration curve and the fluorescence change amount of the donor fluorescent molecules 11 before and after the hybridization reaction using the sample, it is possible to calculate the number of target molecules 30 that have undergone a hybridization reaction.

Similarly, from the fluorescence change amount of the acceptor fluorescent molecules 21 before and after the hybridization reaction, it is also possible to calculate the number of target molecules 30 that have not undergone a hybridization reaction. For example, a hybridization reaction is performed using a standard solution of the target molecules 30 having a known number of molecules, the fluorescence change amount of the acceptor fluorescent molecules 21 before and after the reaction is measured, and a calibration curve showing the relationship between the number of molecules and the fluorescence change amount is created in advance. From this calibration curve and the fluorescence change amount of the acceptor fluorescent molecules 21 before and after the hybridization reaction using the sample, it is possible to calculate the number of target molecules 30 that have not undergone a hybridization reaction.

In addition, a coefficient is set to multiply the fluorescence amount of the acceptor fluorescent molecules 21 so that the fluorescence amount of the acceptor fluorescent molecules 21 when there are no target molecules 30 is equal to the fluorescence amount of the donor fluorescent molecules 11, and a numerical value obtained by multiplying the fluorescence amount of the acceptor fluorescent molecules 21 of the target molecules 30 by the set coefficient is subtracted from the fluorescence amount of the donor fluorescent molecules 11, and thereby a corrected fluorescence amount can be calculated. Using a standard solution containing the target molecules 30 having a known number of molecules, a calibration curve showing the relationship between the number of molecules and the corrected fluorescence amount is created in advance. From this calibration curve and the corrected fluorescence amount during the hybridization reaction using the sample, it is possible to calculate the number of target molecules 30 that have undergone a hybridization reaction.

Next, a nucleic acid sequence measurement apparatus of the present invention will be described.

The nucleic acid sequence measurement apparatus of the present invention includes the nucleic acid sequence measurement device of the present invention, and a fluorescence reading apparatus for measuring a fluorescence amount of the donor fluorescent molecules and a fluorescence amount of the acceptor fluorescent molecules from the nucleic acid sequence measurement device.

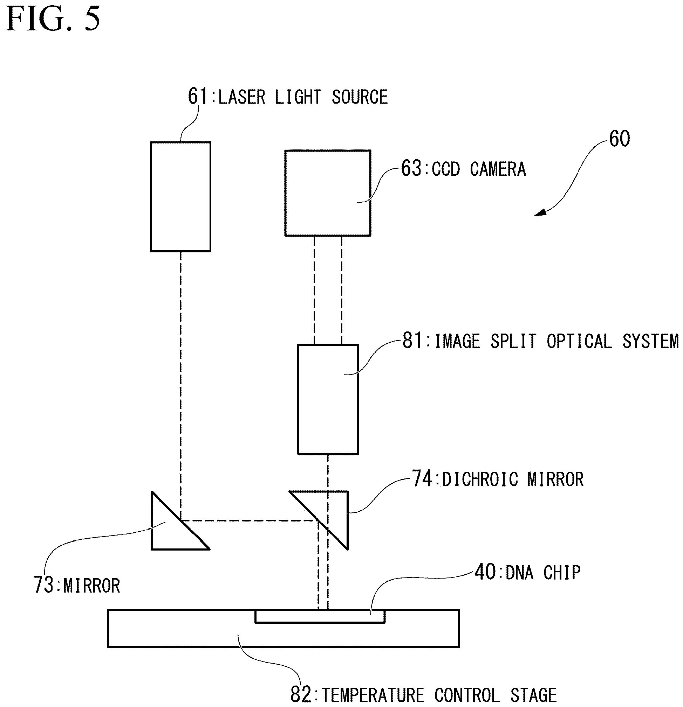

FIG. 5 is a configuration diagram showing the nucleic acid sequence measurement apparatus 60 of the present invention. In order for the nucleic acid sequence measurement apparatus of the present invention to acquire images before and after the target 30 of a DNA chip (nucleic acid sequence measurement device) 40 is hybridized with the donor fluorescent probe 10, after the image before hybridization is acquired, the temperature of the DNA chip 40 is raised by a temperature control stage 82 to proceed the hybridization reaction, and the image after hybridization is acquired when the temperature is lowered to room temperature again.

The temperature control stage 82 preferably has a shaking or rotation function of the DNA chip 40, a stirring function with a vortex mixer or the like during the reaction between the target 30 and the probe in order to promote hybridization between the target 30 and the probe.

In an optical system of the nucleic acid sequence measurement apparatus 60, laser light emitted from a laser light source 61 is reflected by a dichroic mirror 74 via a mirror 73 and is emitted to the DNA chip 40. When the emitted light becomes excitation light for donor fluorescent molecules on the DNA chip 40, and the wavelength of the laser light source 61 and the excitation wavelength of the donor fluorescent molecule 11 overlap, the donor fluorescent molecules 11 are excited.

As described above, when the donor fluorescent probe 10 and the quenching probe 20 are bound, the donor fluorescent molecule 11 is quenched, and the acceptor fluorescent molecules 21 exhibit fluorescence. When the target 30 is present and the binding between the donor fluorescent probe 10 and the quenching probe 20 is released, the donor fluorescent molecules 11 exhibit fluorescence, and the acceptor fluorescent molecules 21 do not exhibit fluorescence.

The fluorescence emitted from the DNA chip 40 passes through the dichroic mirror 74 and separated into two colors via an image split optical system 81 which is an imaging optical system, and two images at the same coordinates separated by wavelength on the detection element of a CCD camera 63 are separately formed and detected. Therefore, it is possible to acquire the images before and after hybridization at the same coordinates and acquire two images separated by wavelength at the same time. In addition, detection is possible with one CCD camera 63.

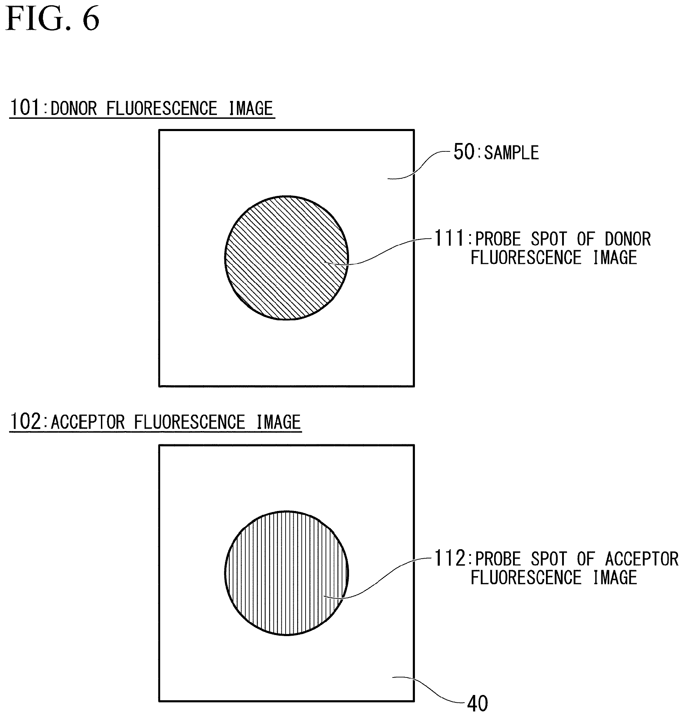

FIG. 6 is a schematic diagram of two CCD camera images separated by wavelength. As shown in FIG. 6, regarding the image obtained by the nucleic acid sequence measurement apparatus of the present invention, images before and after hybridization at the same spot can be acquired. Therefore, it is not affected by variations in the light intensity between chips and between spots.

In addition, since a donor fluorescence image 101 and a fluorescence image (acceptor fluorescence image) 102 from acceptor fluorescent molecules, which are separated by wavelength, can be acquired at the same time, there is no time lag, and the correlation of the images can be corrected.

In addition, it is possible to compute the fluorescence change amount from the donor fluorescence image 101 before and after the hybridization reaction and calculate the number of molecules that have undergone a hybridization reaction, and it is possible to compute the fluorescence change amount from the acceptor fluorescence image 102 before and after the hybridization reaction and calculate the number of molecules that have not undergone a hybridization reaction. The fluorescence change amount may be computed using the average light intensity of the entire spot or using the fluorescence change amount of pixels of the spot image.

The nucleic acid sequence measurement apparatus of the present invention may include a computer that controls the CCD camera 63, an arithmetic apparatus that computes a light intensity of an image, and a recording apparatus that stores the image, the light intensity, and the like.

The scope of application of the present invention is not limited to the above embodiment. The present invention can be widely applied to a nucleic acid sequence measurement device for measuring a target having a specific nucleic acid sequence contained in a sample by hybridization, a nucleic acid sequence measurement method using the nucleic acid sequence measurement device and a nucleic acid sequence measurement apparatus.

### Examples

While the present invention will be described below in more detail with reference to examples, the present invention is not limited to the following examples.

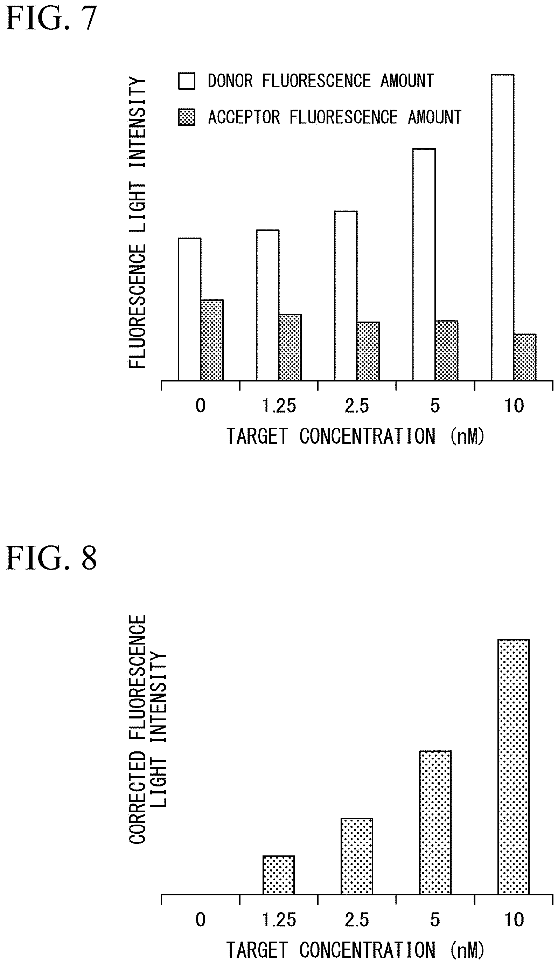

A DNA chip 40 in which a plurality of donor fluorescent probes 10 and quenching probes 20 were arranged on a substrate was prepared, the target 30 was supplied to the DNA chip 40 and reacted at 60°C for 30 minutes, and a fluorescence amount of the donor fluorescent molecules from the DNA chip and a fluorescence amount of the acceptor fluorescent molecules from the quenching probe were measured. The results are shown in FIG. 7. Here, in this example, Cy3 was used as the donor fluorescent molecule 11, and Cy5 was used as the acceptor fluorescent molecule 25.

FIG. 7 is a diagram showing a change in the amount of donor fluorescence which is fluorescence of the donor fluorescent molecules 11 of the donor fluorescent probe 10 and in the amount of acceptor fluorescence which is fluorescence of the acceptor fluorescent molecules 21 of the quenching probe 20 when there is no target 30 and the concentration of the target 30 is increased.

As shown in FIG. 7, the fluorescence amount of the donor fluorescent molecules increases as the concentration of the target 30 increased. In addition, the fluorescence amount of the acceptor fluorescent molecules decreases as the concentration of the target 30 increased.

Next, in FIG. 7, a coefficient was set to multiply the fluorescence amount of the acceptor fluorescent molecules so that the fluorescence amount of the acceptor fluorescent molecules when there was no target 30 (in FIG. 7, when the target concentration was 0 nM) was equal to the fluorescence amount of the donor fluorescent molecules. Then, a numerical value obtained by multiplying the fluorescence amount of the acceptor fluorescent molecules at each concentration of each target 30 by the set coefficient was subtracted from the fluorescence amount of the donor fluorescent molecules, and thereby a corrected light intensity was calculated. The results are shown in FIG. 8. As shown in FIG. 8, it was confirmed that the slope of the increase in the corrected light intensity with respect to the slope of the increase in the fluorescence amount of the donor fluorescent molecules with respect to the concentration of the target 30 increased about 1.4 times, and the sensitivity was improved.

In FIG. 8, the corrected light intensity when there was no corrected target 30 was 0, but the confidence interval of the lower limit of detection was actually determined according to the variation in the correlation between the fluorescence amount of the donor fluorescent molecules and the fluorescence amount of the acceptor fluorescent molecules. It was thought that the correlation between the fluorescence amount of the donor fluorescent molecules and the fluorescence amount of the acceptor fluorescent molecules varied largely in the order of the chip-to-chip difference, the spot-to-spot difference, and the non-uniformity of the amount of the probe fixed in the spot. The nucleic acid sequence measurement apparatus of the present invention removed the influence of the spot-to-spot difference and the amount of the probe fixed in the spot, and could detect the amount of change in the light intensity in pixels of the fluorescence image even with respect to the non-uniformity of the amount of the probe fixed in the spot, selected pixels having a large correlation, and computed a fluorescence light intensity. Therefore, the nucleic acid sequence measurement apparatus of the present invention could reduce the variation to a low level.

While preferable examples of the present invention have been described above, the present invention is not limited to these examples. The present invention is not limited to the above descriptions, but it only limited by the scope of the appended claims.

### [Reference Signs List]

10 Donor fluorescent probe
11 Donor fluorescent molecule
12 Part X (first binding part)
13 Detection sequence (detection part)
14 Linker (first base end)
20 Quenching probe
21 Acceptor fluorescent molecule
22 Part Y (second binding part)
23 Detection sequence (detection part)
24 Linker (second base end)
40 DNA chip (nucleic acid sequence measurement device)
50 Sample
60 Fluorescence reading apparatus (nucleic acid sequence measurement apparatus)
61 Laser light source
63 CCD camera
73 Mirror
74 Dichroic mirror
81 Image split optical system
82 Temperature control stage
100 Solid phase surface
101 Donor fluorescence image
102 Acceptor fluorescence image
111 Probe spot of donor fluorescence image
112 Probe spot of acceptor fluorescence image

## Claims

1. A nucleic acid sequence measurement device (40) that measures a target (30) having a specific nucleic acid sequence included in a sample (50) by means of hybridization, the nucleic acid sequence measurement device (40) comprising:
a donor fluorescent probe (10) having a first binding part (12) and a first base end (14) and having a donor fluorescent molecule (11) added at predetermined position;
a quenching probe (20) having a second binding part (22) and a second base end (24) and having an acceptor fluorescent molecule (21) added at predetermined position; and
a substrate having a solid phase surface (100) to which the first base end (14) of the donor fluorescent probe (10) and the second base end (24) of the quenching probe (20) are fixed,
wherein the first binding part (12) of the donor fluorescent probe (10) and the second binding part (22) of the quenching probe (20) have sequences complementary to each other,
wherein at least one of the donor fluorescent probe (10) and the quenching probe (20) has a detection part (13, 23) having a sequence complementary to a nucleic acid sequence of the target (30), and
wherein the donor fluorescent probe (10) and the quenching probe (20) have a positional relationship in which fluorescence of the donor fluorescent molecule (11) is quenched with the acceptor fluorescent molecule (21) that approaches the donor fluorescent molecule (11), and the first base end (14) and the second base end (24) are fixed to the solid phase surface (100).

2. The nucleic acid sequence measurement device (40) according to claim 1,
wherein, when hybridization between the target (30) and the detection part (13, 23) has not occurred, a binding between the first binding part (12) of the donor fluorescent probe (10) and the quenching probe (20) is maintained, and thus the fluorescence of the donor fluorescent molecule (11) is quenched with the acceptor fluorescent molecule (21) that approaches the donor fluorescent molecule (11), and the acceptor fluorescent molecule (21) exhibits fluorescence, and
wherein, when hybridization between the target (30) and the detection part (13, 23) occurs, the binding between the first binding part (12) of the donor fluorescent probe (10) and the second binding part (22) of the quenching probe (20) is released, the donor fluorescent molecule (11) separated from the acceptor fluorescent molecule (21) exhibits fluorescence.

3. The nucleic acid sequence measurement device (40) according to claim 1 or 2, wherein the donor fluorescent probe (10) has the detection part (13).

4. The nucleic acid sequence measurement device (40) according to claim 1 or 2, wherein the quenching probe (20) has the detection part (23).

5. The nucleic acid sequence measurement device (40) according to any one of claims 1 to 4, wherein the substrate is a flat plate, and the solid phase surface (100) is a flat surface of the flat plate.

6. The nucleic acid sequence measurement device (40) according to any one of claims 1 to 3 and 5, wherein at least a part of the first binding part (12) of the donor fluorescent probe (10) functions as the detection part (13).

7. The nucleic acid sequence measurement device (40) according to any one of claims 1, 2, 4 and 5, wherein at least a part of the second binding part (22) of the quenching probe (20) functions as the detection part (23).

8. The nucleic acid sequence measurement device (40) according to any one of claims 1 to 7, wherein the predetermined position to which the donor fluorescent molecule (11) is added is in the middle of the donor fluorescent probe (10).

9. The nucleic acid sequence measurement device (40) according to any one of claims 1 to 7, wherein the predetermined position to which the acceptor fluorescent molecule (21) is added is in the middle of the quenching probe (20).

10. The nucleic acid sequence measurement device (40) according to any one of claims 1 to 9, wherein both the donor fluorescent probe (10) and the quenching probe (20) have the detection part (13, 23).

11. A nucleic acid sequence measurement method of measuring a target (30) having a specific nucleic acid sequence included in a sample (50) by means of hybridization, the nucleic acid sequence measurement method comprising:
(a) a step of preparing a sample (50) containing the target (30);
(b) a step of supplying the sample (50) to a nucleic acid sequence measurement device (40);
(c) a step of measuring a fluorescence amount of a donor fluorescent molecule (11) and a fluorescence amount of an acceptor fluorescent molecule (21) from the nucleic acid sequence measurement device (40);
(d) a step of causing a hybridization reaction between the target (30) in the sample (50) and at least one of a donor fluorescent probe (10) or a quenching probe (20) in the nucleic acid sequence measurement device (40); and
(e) a step of measuring a fluorescence amount of the donor fluorescent molecule (11) and a fluorescence amount of the acceptor fluorescent molecule (21) from the nucleic acid sequence measurement device (40) after the reaction,
wherein the nucleic acid sequence measurement device (40) comprises:
a donor fluorescent probe (10) having a first binding part (12) and a first base end (14) and having the donor fluorescent molecule (11) added at a predetermined position;
a quenching probe (20) having a second binding part (22) and a second base end (24) and having the acceptor fluorescent molecule (21) added at a predetermined position; and
a substrate having a solid phase surface (100) to which the first base end (14) of the donor fluorescent probe (10) and the second base end (24) of the quenching probe (20) are fixed,
wherein the first binding part (12) of the donor fluorescent probe (10) and the second binding part (22) of the quenching probe (20) have sequences complementary to each other,
wherein at least one of the donor fluorescent probe (10) and the quenching probe (20) has a detection part (13, 23) having a sequence complementary to a nucleic acid sequence of the target (30), and
wherein the donor fluorescent probe (10) and the quenching probe (20) have a positional relationship in which fluorescence of the donor fluorescent molecule (11) is quenched with the acceptor fluorescent molecule (21) that approaches the donor fluorescent molecule (11), and the first base end (14) and the second base end (24) are fixed to the solid phase surface (100).

12. The nucleic acid sequence measurement method according to claim 11, further comprising:
calculating a number of molecules of the hybridized target (30) from a change in the fluorescence amount of the donor fluorescent molecule (11) between before and after the hybridization reaction.

13. The nucleic acid sequence measurement method according to claim 11, further comprising:
calculating a number of molecules of the target (30) that are not hybridized from a change in the fluorescence amount of the acceptor fluorescent molecule (21) between before and after the hybridization reaction.

14. A nucleic acid sequence measurement apparatus comprising:
the nucleic acid sequence measurement device (40) according to any one of claims 1 to 10; and
a fluorescence reading apparatus (60) configured to measure a fluorescence amount of the donor fluorescent molecule (11) and a fluorescence amount of the acceptor fluorescent molecule (21) from the nucleic acid sequence measurement device (40).

15. The nucleic acid sequence measurement apparatus according to claim 14, wherein, in a presence of the sample (50), a fluorescence amount of the donor fluorescent molecule (11) and a fluorescence amount of the acceptor fluorescent molecule (21) from the nucleic acid sequence measurement device (40) are measured.

16. The nucleic acid sequence measurement apparatus according to claim 14 or claim 15, further comprising a stirring function for hybridizing the target (30) with the donor fluorescent probe (10) or the quenching probe (20).

17. The nucleic acid sequence measurement apparatus according to any one of claims 14 to 16, further comprising a function of separating the fluorescence from the nucleic acid sequence measurement device (40) into two colors and measuring the fluorescence at the same time.

## Patentansprüche

1. Nukleinsäuresequenz-Messeinrichtung (40), die ein Ziel (30) mit einer spezifischen Nukleinsäuresequenz, das in einer Probe (50) enthalten ist, mittels Hybridisierung misst, wobei die Nukleinsäuresequenz-Messeinrichtung (40) umfasst:
eine Donorfluoreszenz-Sonde (10) mit einem ersten Bindungsteil (12) und einem ersten Basen-Ende (14), die ein an einer vorgegebenen Position hinzugefügtes Donorfluoreszenz-Molekül (11) aufweist;
eine Quenching-Sonde (20) mit einem zweiten Bindungsteil (22) und einem zweiten Basen-Ende (24), die ein an einer vorgegebenen Position hinzugefügtes Akzeptorfluoreszenz-Molekül (21) aufweist;
ein Substrat mit einer Festphasen-Fläche (100), an der das erste Basen-Ende (14) der Donorfluoreszenz-Sonde (10) und das zweite Basen-Ende (24) der Quenching-Sonde (20) fixiert sind,
wobei der erste Bindungsteil (12) der Donorfluoreszenz-Sonde (10) und der zweite Bindungsteil (22) der Quenching-Sonde (20) zueinander komplementäre Sequenzen aufweisen,
wobei die Donorfluoreszenz-Sonde (10) oder/und die Quenching-Sonde (20) einen Erfassungsteil (13, 23) mit einer Sequenz aufweist/aufweisen, die komplementär zu einer Nukleinsäuresequenz des Objektes (30) ist, und
die Donorfluoreszenz-Sonde (10) und die Quenching-Sonde (20) eine Positionsbeziehung haben, in der Fluoreszenz des Donorfluoreszenz-Moleküls (11) mit dem Akzeptorfluoreszenz-Molekül (21) gelöscht wird, das sich dem Donorfluoreszenz-Molekül (11) nähert, und das erste Basen-Ende (14) sowie das zweite Basen-Ende (24) an der Festphasen-Fläche (100) fixiert sind.

2. Nukleinsäuresequenz-Messeinrichtung (40) nach Anspruch 1,
wobei, wenn Hybridisierung zwischen dem Ziel (30) und dem Erfassungsteil (13, 23) nicht aufgetreten ist, eine Bindung zwischen dem ersten Bindungsteil (12) der Donorfluoreszenz-Sonde (10) und der Quenching-Sonde (20) aufrechterhalten wird und damit die Fluoreszenz des Donorfluoreszenz-Moleküls (11) mit dem Akzeptorfluoreszenz-Molekül (21) gelöscht wird, das sich dem Donorfluoreszenz-Molekül (11) nähert, und das Akzeptorfluoreszenz-Molekül (21) Fluoreszenz aufweist, und
wobei, wenn eine Hybridisierung zwischen dem Ziel (30) und dem Erfassungsteil (13, 23) auftritt, die Bindung zwischen dem ersten Bindungsteil (12) der Donorfluoreszenz-Sonde (10) und dem zweiten Bindungsteil (22) der Quenching-Sonde (20) gelöst wird, das von dem Akzeptorfluoreszenz-Molekül (21) getrennte Donorfluoreszenz-Molekül (11) Fluoreszenz aufweist.

3. Nukleinsäuresequenz-Messeinrichtung (40) nach Anspruch 1 oder 2, wobei die Donorfluoreszenz-Sonde (10) den Erfassungsteil (13) aufweist.

4. Nukleinsäuresequenz-Messeinrichtung (40) nach Anspruch 1 oder 2, wobei die Quenching-Sonde (20) den Erfassungsteil (23) aufweist.

5. Nukleinsäuresequenz-Messeinrichtung (40) nach einem der Ansprüche 1 bis 4, wobei das Substrat eine flache Platte ist und die Festphasen-Fläche (100) eine plane Fläche der flachen Platte ist.

6. Nukleinsäuresequenz-Messeinrichtung (40) nach einem der Ansprüche 1 bis 3 sowie 5, wobei wenigstens ein Teil des ersten Bindungsteils (12) der Donorfluoreszenz-Sonde (10) als der Erfassungsteil (13) fungiert.

7. Nukleinsäuresequenz-Messeinrichtung (40) nach einem der Ansprüche 1, 2, 4 sowie 5, wobei wenigstens ein Teil des zweiten Bindungsteils (22) der Quenching-Sonde (20) als der Erfassungsteil (23) fungiert.

8. Nukleinsäuresequenz-Messeinrichtung (40) nach einem der Ansprüche 1 bis 7, wobei die vorgegebene Position, an der das Donorfluoreszenz-Molekül (11) hinzugefügt wird, in der Mitte der Donorfluoreszenz-Sonde (10) liegt.

9. Nukleinsäuresequenz-Messeinrichtung (40) nach einem der Ansprüche 1 bis 7, wobei die vorgegebene Position, an der das Akzeptorfluoreszenz-Molekül (21) hinzugefügt wird, in der Mitte der Quenching-Sonde (20) liegt.

10. Nukleinsäuresequenz-Messeinrichtung (40) nach einem der Ansprüche 1 bis 9, wobei sowohl die Donorfluoreszenz-Sonde (10) als auch die Quenching-Sonde (20) den Erfassungsteil (13, 23) aufweisen.

11. Nukleinsäuresequenz-Messverfahren (40) zum Messen eines Ziels (30) mit einer spezifischen Nukleinsäuresequenz, die in einer Probe (50) enthalten ist, mittels Hybridisierung, wobei das Nukleinsäuresequenz-Messverfahren (40) umfasst:
(a) einen Schritt zum Fertigen einer Probe (50), die das Ziel (30) enthält;
(b) einen Schritt zum Zuführen der Probe (50) zu einer Nukleinsäuresequenz-Messeinrichtung (40);
(c) einen Schritt zum Messen einer Fluoreszenzmenge eines Donorfluoreszenz-Moleküls (11) und einer Fluoreszenzmenge eines Akzeptorfluoreszenz-Moleküls (21) von der Nukleinsäuresequenz-Messeinrichtung (40);
(d) einen Schritt zum Auslösen einer Hybridisierungs-Reaktion zwischen dem Ziel (30) in der Probe (50) und einer Donorfluoreszenz-Sonde (10) oder/und einer Quenching-Sonde (20) in der Nukleinsäuresequenz-Messeinrichtung (40); sowie
(e) einen Schritt zum Messen einer Fluoreszenzmenge des Donorfluoreszenz-Moleküls (11) und einer Fluoreszenzmenge des Akzeptorfluoreszenz-Moleküls (21) von der Nukleinsäuresequenz-Messeinrichtung (40) nach der Reaktion;
wobei die Nukleinsäuresequenz-Messeinrichtung (40) umfasst:
eine Donorfluoreszenz-Sonde (10) mit einem ersten Bindungsteil (12) und einem ersten Basen-Ende (14), die das an einer vorgegebenen Position hinzugefügte Donorfluoreszenz-Molekül (11) aufweist;
eine Quenching-Sonde (20) mit einem zweiten Bindungsteil (22) und einem zweiten Basen-Ende (24), die das an einer vorgegebenen Position hinzugefügte Akzeptorfluoreszenz-Molekül (21) aufweist;
ein Substrat mit einer Festphasen-Fläche (100), an der das erste Basen-Ende (14) der Donorfluoreszenz-Sonde (10) und das zweite Basen-Ende (24) der Quenching-Sonde (20) fixiert sind,
wobei der erste Bindungsteil (12) der Donorfluoreszenz-Sonde (10) und der zweite Bindungsteil (22) der Quenching-Sonde (20) zueinander komplementäre Sequenzen aufweisen,
wobei die Donorfluoreszenz-Sonde (10) oder/und die Quenching-Sonde (20) einen Erfassungsteil (13, 23) mit einer Sequenz aufweist/aufweisen, die komplementär zu einer Nukleinsäuresequenz des Objektes (30) ist, und
die Donorfluoreszenz-Sonde (10) und die Quenching-Sonde (20) eine Positionsbeziehung haben, in der Fluoreszenz des Donorfluoreszenz-Moleküls (11) mit dem Akzeptorfluoreszenz-Molekül (21) gelöscht wird, das sich dem Donorfluoreszenz-Molekül (11) nähert, und das erste Basen-Ende (14) sowie das zweite Basen-Ende (24) an der Festphasen-Fläche (100) fixiert sind.

12. Nukleinsäuresequenz-Messverfahren nach Anspruch 11, das des Weiteren umfasst:
Berechnen einer Anzahl von Molekülen des hybridisierten Ziels (30) anhand einer Änderung der Fluoreszenzmenge des Donorfluoreszenz-Moleküls (11) zwischen der Zeit vor der Hybridisierungs-Reaktion und der Zeit danach.

13. Nukleinsäuresequenz-Messverfahren nach Anspruch 11, das des Weiteren umfasst:
Berechnen einer Anzahl von Molekülen des Ziels (30), die nicht hybridisiert sind, anhand einer Änderung der Fluoreszenzmenge des Akzeptorfluoreszenz-Moleküls (21) zwischen der Zeit vor der Hybridisierungs-Reaktion und der Zeit danach.

14. Nukleinsäuresequenz-Messvorrichtung, die umfasst:
die Nukleinsäuresequenz-Messeinrichtung (40) nach einem der Ansprüche 1 bis 10 sowie
eine Fluoreszenz-Messvorichtung (60), die so ausgeführt ist, dass sie eine Fluoreszenzmenge des Donorfluoreszenz-Moleküls (11) und eine Fluoreszenzmenge des Akzeptorfluoreszenz-Moleküls (21) von der Nukleinsäuresequenz-Messeinrichtung (40) misst.

15. Nukleinsäuresequenz-Messvorrichtung nach Anspruch 14, wobei beim Vorhandensein der Probe (50) eine Fluoreszenzmenge des Donorfluoreszenz-Moleküls (11) und eine Fluoreszenzmenge des Akzeptorfluoreszenz-Moleküls (21) von der Nukleinsäuresequenz-Messeinrichtung (40) gemessen werden.

16. Nukleinsäuresequenz-Messvorrichtung nach Anspruch 14 oder Anspruch 15, die des Weiteren eine Rühr-Funktion zum Hybridisieren des Ziels (30) mit der Donorfluoreszenz-Sonde (10) oder der Quenching-Sonde (20) umfasst.

17. Nukleinsäuresequenz-Messvorrichtung nach einem der Ansprüche 14 bis 16, die des Weiteren eine Funktion zum Trennen der Fluoreszenz von der Nukleinsäuresequenz-Messeinrichtung (40) in zwei Farben und gleichzeitigen Messen der Fluoreszenz umfasst.

## Revendications

1. Dispositif (40) de mesure de séquence d'acide nucléique qui mesure une cible (30) ayant une séquence d'acide nucléique spécifique incluse dans un échantillon (50) au moyen d'une hybridation, le dispositif (40) de mesure de séquence d'acide nucléique comprenant :
une sonde fluorescente donneuse (10) ayant une première partie de liaison (12) et une première extrémité de base (14) et ayant une molécule fluorescente donneuse (11) ajoutée à une position prédéfinie ;
une sonde d'extinction (20) ayant une seconde partie de liaison (22) et une seconde extrémité de base (24) et ayant une molécule fluorescente acceptrice (21) ajoutée à une position prédéfinie ; et
un substrat ayant une surface solide (100) sur laquelle sont fixées la première extrémité de base (14) de la sonde fluorescente donneuse (10) et la seconde extrémité de base (24) de la sonde d'extinction (20),
dans lequel la première partie de liaison (12) de la sonde fluorescente donneuse (10) et la seconde partie de liaison (22) de la sonde d'extinction (20) ont des séquences complémentaires l'une de l'autre,
dans lequel l'une au moins de la sonde fluorescente donneuse (10) et de la sonde d'extinction (20) possède une partie de détection (13, 23) ayant une séquence complémentaire d'une séquence d'acide nucléique de la cible (30), et
dans lequel la sonde fluorescente donneuse (10) et la sonde d'extinction (20) ont une relation de position dans laquelle la fluorescence de la molécule fluorescente donneuse (11) est éteinte par la molécule fluorescente acceptrice (21) qui s'approche de la molécule fluorescente donneuse (11), et la première extrémité de base (14) et la seconde extrémité de base (24) sont fixées sur la surface solide (100).

2. Dispositif (40) de mesure de séquence d'acide nucléique selon la revendication 1,
dans lequel, lorsque l'hybridation entre la cible (30) et la partie de détection (13, 23) ne s'est pas produite, une liaison entre la première partie de liaison (12) de la sonde fluorescente donneuse (10) et la sonde d'extinction (20) est maintenue, en conséquence de quoi la fluorescence de la molécule fluorescente donneuse (11) est éteinte par la molécule fluorescente acceptrice (21) qui s'approche de la molécule fluorescente donneuse (11), et la molécule fluorescente acceptrice (21) présente une fluorescence, et
dans lequel, lorsque l'hybridation entre la cible (30) et la partie de détection (13, 23) se produit, la liaison entre la première partie de liaison (12) de la sonde fluorescente donneuse (10) et la seconde partie de liaison (22) de la sonde d'extinction (20) est déliée, la molécule fluorescente donneuse (11) séparée de la molécule fluorescente acceptrice (21) présentant une fluorescence.

3. Dispositif (40) de mesure de séquence d'acide nucléique selon la revendication 1 ou 2, dans lequel la sonde fluorescente donneuse (10) possède la partie de détection (13).

4. Dispositif de mesure de séquence d'acide nucléique (40) selon la revendication 1 ou 2, dans lequel la sonde d'extinction (20) possède la partie de détection (23).

5. Dispositif (40) de mesure de séquence d'acide nucléique selon l'une quelconque des revendications 1 à 4, dans lequel le substrat est une plaque plane, et la surface solide (100) est une surface plane de la plaque plane.

6. Dispositif (40) de mesure de séquence d'acide nucléique selon l'une quelconque des revendications 1 à 3 et 5, dans lequel une partie au moins de la première partie de liaison (12) de la sonde fluorescente donneuse (10) fonctionne en tant que partie de détection (13).

7. Dispositif (40) de mesure de séquence d'acide nucléique selon l'une quelconque des revendications 1, 2, 4 et 5, dans lequel une partie au moins de la seconde partie de liaison (22) de la sonde d'extinction (20) fonctionne en tant que partie de détection (23).

8. Dispositif (40) de mesure de séquence d'acide nucléique selon l'une quelconque des revendications 1 à 7, dans lequel la position prédéfinie à laquelle est ajoutée la molécule fluorescente donneuse (11) est au milieu de la sonde fluorescente donneuse (10).

9. Dispositif (40) de mesure de séquence d'acide nucléique selon l'une quelconque des revendications 1 à 7, dans lequel la position prédéfinie à laquelle est ajoutée la molécule fluorescente acceptrice (21) est au milieu de la sonde d'extinction (20).

10. Dispositif (40) de mesure de séquence d'acide nucléique selon l'une quelconque des revendications 1 à 9, dans lequel la sonde fluorescente donneuse (10) et la sonde d'extinction (20) possèdent toutes les deux la partie de détection (13, 23).

11. Procédé de mesure de séquence d'acide nucléique consistant à mesurer une cible (30) ayant une séquence d'acide nucléique spécifique incluse dans un échantillon (50) au moyen d'une hybridation, le procédé de mesure de séquence d'acide nucléique comprenant :
(a) une étape consistant à préparer un échantillon (50) contenant la cible (30) ;
(b) une étape consistant à fournir l'échantillon (50) à un dispositif (40) de mesure de séquence d'acide nucléique ;
(c) une étape consistant à mesurer une quantité de fluorescence d'une molécule fluorescente donneuse (11) et une quantité de fluorescence d'une molécule fluorescente acceptrice (21) du dispositif (40) de mesure de séquence d'acide nucléique ;
(d) une étape consistant à provoquer une réaction d'hybridation entre la cible (30) dans l'échantillon (50) et l'une au moins d'une sonde fluorescente donneuse (10) ou d'une sonde d'extinction (20) dans le dispositif (40) de mesure de séquence d'acide nucléique ; et
(e) une étape de mesure d'une quantité de fluorescence de la molécule fluorescente donneuse (11) et d'une quantité de fluorescence de la molécule fluorescente acceptrice (21) par le dispositif (40) de mesure de séquence d'acide nucléique après la réaction,
le dispositif (40) de mesure de séquence d'acide nucléique comprenant :
une sonde fluorescente donneuse (10) ayant une première partie de liaison (12) et une première extrémité de base (14) et ayant la molécule fluorescente donneuse (11) ajoutée à une position prédéfinie ;
une sonde d'extinction (20) ayant une seconde partie de liaison (22) et une seconde extrémité de base (24) et ayant la molécule fluorescente acceptrice (21) ajoutée à une position prédéfinie ; et
un substrat ayant une surface solide (100) sur laquelle sont fixées la première extrémité de base (14) de la sonde fluorescente donneuse (10) et la seconde extrémité de base (24) de la sonde d'extinction (20),
dans lequel la première partie de liaison (12) de la sonde fluorescente donneuse (10) et la seconde partie de liaison (22) de la sonde d'extinction (20) ont des séquences complémentaires l'une de l'autre,
dans lequel l'une au moins de la sonde fluorescente donneuse (10) et de la sonde d'extinction (20) possède une partie de détection (13, 23) ayant une séquence complémentaire d'une séquence d'acide nucléique de la cible (30), et
dans lequel la sonde fluorescente donneuse (10) et la sonde d'extinction (20) ont une relation de position dans laquelle la fluorescence de la molécule fluorescente donneuse (11) est éteinte par la molécule fluorescente acceptrice (21) qui s'approche de la molécule fluorescente donneuse (11), et la première extrémité de base (14) et la seconde extrémité de base (24) sont fixées sur la surface solide (100).

12. Procédé de mesure de séquence d'acide nucléique selon la revendication 11, comprenant en outre :
le calcul d'un nombre de molécules de la cible (30) hybridée à partir d'un changement de la quantité de fluorescence de la molécule fluorescente donneuse (11) entre avant et après la réaction d'hybridation.

13. Procédé de mesure de séquence d'acide nucléique selon la revendication 11, comprenant en outre :
le calcul d'un nombre de molécules de la cible (30) qui ne sont pas hybridées à partir d'un changement de la quantité de fluorescence de la molécule fluorescente acceptrice (21) entre avant et après la réaction d'hybridation.

14. Appareil de mesure de séquence d'acide nucléique comprenant :
le dispositif (40) de mesure de séquence d'acide nucléique selon l'une quelconque des revendications 1 à 10 ; et
un appareil de lecture de fluorescence (60) configuré pour mesurer une quantité de fluorescence de la molécule fluorescente donneuse (11) et une quantité de fluorescence de la molécule fluorescente acceptrice (21) du dispositif (40) de mesure de séquence d'acide nucléique.

15. Appareil de mesure de séquence d'acide nucléique selon la revendication 14, dans lequel, en présence de l'échantillon (50), une quantité de fluorescence de la molécule fluorescente donneuse (11) et une quantité de fluorescence de la molécule fluorescente acceptrice (21) du dispositif (40) de mesure de séquence d'acide nucléique sont mesurées.

16. Appareil de mesure de séquence d'acide nucléique selon la revendication 14 ou la revendication 15, comprenant en outre une fonction d'agitation pour hybrider la cible (30) avec la sonde fluorescente donneuse (10) ou la sonde d'extinction (20).

17. Appareil de mesure de séquence d'acide nucléique selon l'une quelconque des revendications 14 à 16, comprenant en outre une fonction consistant à séparer la fluorescence provenant du dispositif (40) de mesure de séquence d'acide nucléique en deux couleurs et à mesurer en même temps la fluorescence.
